# EUROPÄISCHE PATENTSCHRIFT

(11) **EP 0 857 041 B1**
(45) Veröffentlichungstag und Bekanntmachung des Hinweises auf die Patenterteilung: **11.04.2001**
(21) Anmeldenummer: 95934030.8
(22) Anmeldetag: 20.10.1995
(51) Int. Cl.: A61F 2/44

(54) **ZWISCHENWIRBEL-IMPLANTAT MIT KÄFIG UND ROTATIONSKÖRPER**
INTERVERTEBRAL IMPLANT WITH CAGE AND ROTATING ELEMENT
IMPLANT INTERVERTEBRAL AVEC CAGE ET CORPS DE ROTATION

(43) Veröffentlichungstag der Anmeldung: 12.08.1998
(73) Patentinhaber: SYNTHES AG Chur, 7002 Chur (CH)
(72) Erfinder: KNOTHE, Inga, CH-2503 Biel (CH); BENOIT, Alfred, CH-2543 Lengnau (CH)
(74) Vertreter: Lusuardi, Werther Giovanni, Dr.
(86) Internationale Anmeldenummer: CH9500243
(87) Internationale Veröffentlichungsnummer: WO9715246

(56) Entgegenhaltungen:
- WO-A-89/09035
- WO-A-91/06261
- WO-A-94/17759
- US-A- 5 192 327

## Beschreibung

Die Erfindung betrifft ein Zwischenwirbel-Implantat gemäss dem Oberbegriff des Patentanspruchs 1, wie es beispielsweise aus der WO-A-89 09 035 bekannt ist.

Solche Zwischenwirbel-Implantate werden bei der Fusion von Wirbelkörpern eingesetzt, insbesondere im Bereich der lumbalen Wirbelsäule. Pro Zwischenwirbelraum werden ein bis zwei Implantate verwendet.

Aus dem Stand der Technik sind bereits verschiedene Typen derartiger Zwischenwirbel-Implantate bekannt, welche allerdings folgende Nachteile aufweisen:
- um das Implantat in den Zwischenwirbelbereich einführen zu können müssen die betroffenen Wirbel mit geeigneten Instrumenten distrahiert werden; und
- es besteht die Gefahr des Einsinkens der Implantate in die Endplatten der betroffenen Wirbel.

Hier will die Erfindung Abhilfe schaffen. Der Erfindung liegt das Problem zugrunde, ein Zwischenwirbel-Implantat zu schaffen, welches möglichst ohne Distraktionsinstrument in kontrollierbarer Weise mit minimalem Kraftaufwand in den ausgeräumten Zwischenwirbelraum eindrehbar ist.

Zur Lösung dieses Problems ist die eingangs genannte Anordnung durch die Merkmale des kennzeichnenden Teils des unabhängigen Anspruchs 1 weitergebildet.

Damit ist der Vorteil erzielbar, dass ein minimaler Kraftaufwand für die Implantation genügt ist und ein kontrolliertes Einfügen des Implantates mittels der über die Deck- und Grundfläche aus dem Käfig herausragenden spiralförmigen Struktur der Mantelfläche des Rotationskörpers möglich ist. Eine minimalinvasive und offene Operationstechnik ist damit anwendbar.
Die käfigartige Rahmenstruktur mit seiner grossen Auflagefläche verhindert ein Einsinken des Implantates in die Endplatten.

Die über dem Käfig vorstehende spiralförmige Struktur des Rotationskörpers erlaubt es, das Implantat während dessen Einführung zu drehen und in den Zwischenwirbelraum zu schrauben.

Eine bevorzugte Weiterbildung besteht darin, dass der Rotationskörper des Implantats hohl ausgebildet ist und an seiner Frontfläche mit einen Verschlussdeckel versehen ist. Knochenspäne oder Knochenersatzmaterialien können dadurch leicht in den Rotationskörper eingefüllt werden und das Implantat mit wenigen Handgriffen sicher montiert werden. Der Käfig ist in diesem Falle zweckmässigerweise zweiteilig zusammensetzbar ausgebildet, um die Montage zu ermöglichen. Bei dieser Anwendungsform ist der Rotationskörper auch vorzugsweise mit Perforationen in Form von Längsnuten und die Seitenflächen des Käfigs mit Langlochausnehmungen versehen, um ein rasches Einwachsen des Knochens zu ermöglichen.
Die Längsnuten erlauben eine Kontrolle des Knocheneinwachsens mittels Röntgenaufnahmen. Vorzugsweise sind die Längsausnehmungen mit einer Schneidkante versehen, welche die Endplattenspäne in den hohlen Rotationskörper eindringen lassen.

Der Käfig kann quaderförmig ausgebildet sein mit einem kreiszylinderförmigen Rotationskörper, oder keilförmig mit einem entsprechend konisch ausgebildeten Rotationskörper.

Zur Erhöhung der Lagestabilität des Implantates, bzw. der Rotationsstabilität der benachbarten Wirbelkörper ist die Deck- und Grundfläche des Käfigs zweckmässigerweise mit einer dreidimensionalen Strukturierung, vorzugsweise in Form von Längsrillen versehen.
Folgende weitere Vorteile des erfindungsgemässen Implantats ergeben sich gegenüber dem Stand der Technik:
- Verrutschsicherheit;
- verbesserte Röntgendurchlässigkeit; und
- Kompressibilität von allfällig im Rotationskörper eingeführtem Knochenmaterial.

Die Erfindung und Weiterbildungen der Erfindung werden im folgenden anhand der teilweise schematischen Darstellungen eines Ausführungsbeispiels noch näher erläutert.
Es zeigen:
Fig. 1 eine perspektivische Darstellung des Käfigs des erfindungsgemässen Implantats;
Fig. 2 eine perspektivische Darstellung des geöffneten Rotationskörpers des erfindungsgemässen Implantats;
Fig. 3 eine perspektivische Darstellung des geschlossenen Rotationskörpers des erfindungsgemässen Implantats;
Fig. 4 eine perspektivische Darstellung des vollständig montierten, erfindungsgemässen Implantats;
Fig. 5 eine Seitenansicht des Implantats nach Fig. 4;
Fig. 6 eine Aufsicht des Implantats nach Fig. 4;
Fig. 7 eine perspektivische Darstellung einer weiteren Ausführungsform des erfindungsgemässen Implantats im zerlegten Zustand; und
Fig. 8 eine Seitenansicht des Implantats nach Fig. 7 im montierten Zustand.

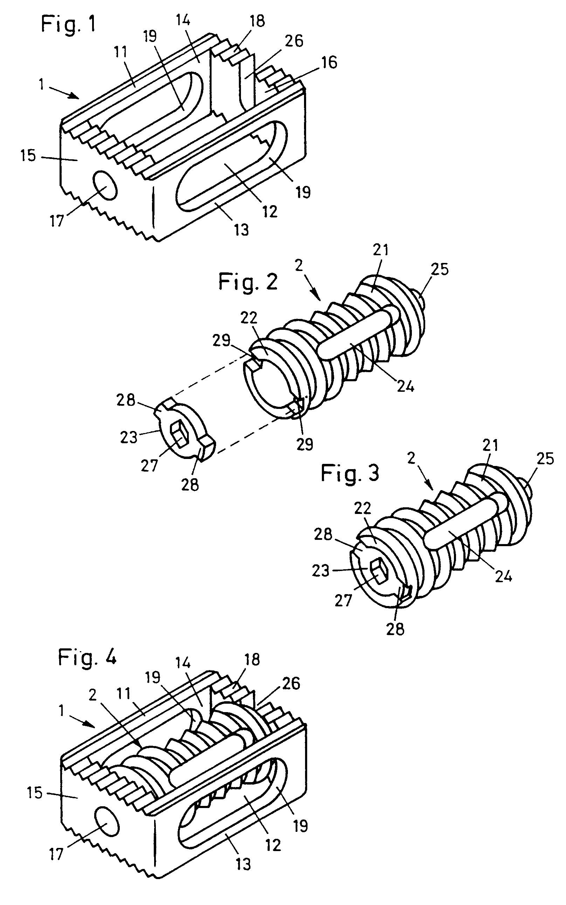

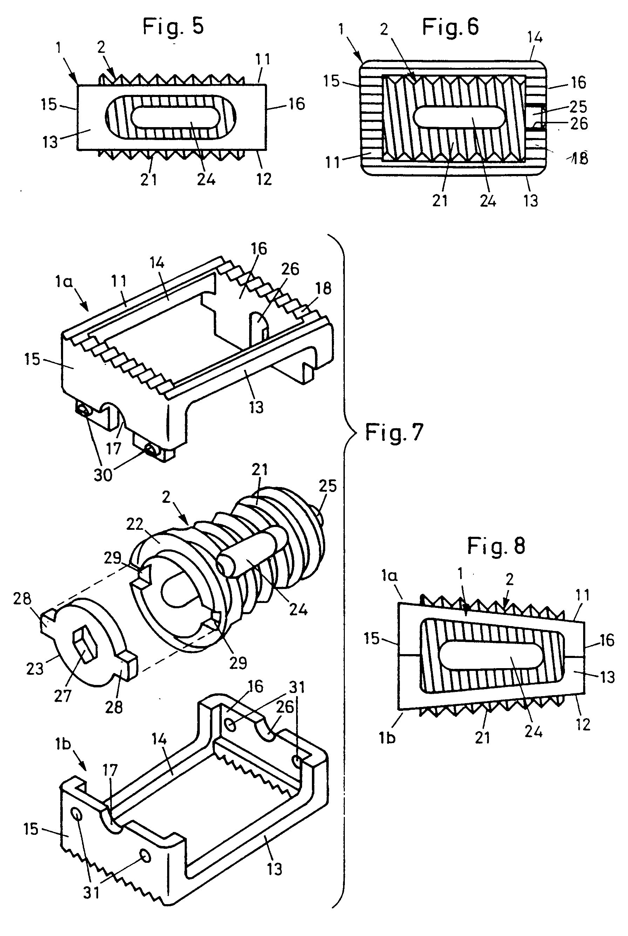

Das in den Fig. 1 - 3 im zerlegten Zustand dargestellte Zwischenwirbel-Implantat besteht im wesentlichen aus einem an seiner Deckfläche 11 und Grundfläche 12 offenen, rahmenförmigen Käfig 1 mit zwei je ein Langloch 19 aufweisenden Seitenflächen 13 und 14, einer, eine Bohrung 17 aufweisenden Frontwand 15 und einer Hinterwand 16. Die Form des Käfigs 1 ist bei dieser Ausführungsform quaderförmig, sie kann jedoch - wie anhand der Fig. 7 und 8 dargestellt - auch keilförmig sein. Der Käfig 1 kann entweder einteilig ausgeführt werden, wie in Fig. 1 dargestellt oder auch mehrteilig, vorzugsweise zweiteilig, wie anhand der Fig. 7 und 8 erläutert.
Die Deck- und Grundfläche 11,12 ist mit einer dreidimensionalen Strukturierung 18, vorzugsweise in Form von Längsrillen versehen, um die Einführung des Implantates und die Rotationsstabilität der benachbarten Wirbelkörper zu verbessern.

Ein hohler, kreiszylindrischer Rotationskörper 2 ist drehbar im Käfig 1 gelagert. Zu diesem Zweck weist der Rotationskörper 2 an seiner hinteren Grundfläche einen Zapfen 25 auf, welcher in die Ausnehmung 26 in der Hinterwand 16 des Käfigs 1 einführbar ist. Die Mantelfläche des Rotationskörpers 2 weist eine spiralförmige Struktur 21 in Form eines Aussengewindes auf, mit einer Steigung von mindestens 2 mm und einer Tiefe von 0,8 - 2,2 mm, vorzugsweise von 1,5 - 2,0 mm.

Die Länge des Rotationskörper 2 ist derart auf die Innendimensionen des Käfig abgestimmt, dass er satt von oben in den Käfig einführbar ist, so dass er ohne weitere Haltemittel im Käfig drehbar gelagert eingeschlossen ist (Fig. 4 und 6). Der Rotationskörper 2 ist an seiner Frontfläche 22 mit einem abnehmbaren Verschlussdeckel 23 mit einer zur Aufnahme eines Antriebswerkzeuges dienenden Sechskantöffnung 27 versehen, damit Knochenspäne oder Knochenersatzmaterial eingefüllt werden können. Der Verschlussdeckel 23 ist mit mindestens zwei gegenüberliegenden Nocken 28 versehen, welche in die entsprechenden Nuten 29 an der Frontfläche 22 des Rotationskörper 2 einrastbar sind, so dass der Verschlussdeckel 23 bündig in der Frontfläche 22 versenkbar ist. Im weiteren ist der Rotationskörper 2 mit Perforationen 24 versehen, welche vorzugsweise als Längsausnehmungen mit einer Schneidkante ausgebildet sind. Die Schneidkante ist vorzugsweise hinterschnitten.

Die Frontwand 15 des Käfigs 1 ist - wie in Fig. 4 ersichtlich - mit einer Bohrung 17 zur Einführung eines Instrumentes in die Sechskantöffnung 27 des Verschlussdeckels 23 versehen, damit der Rotationskörper 2 im Käfig 1 rotiert werden kann.

Wie in Fig. 5 ersichtlich, ragt die sprialförmige Struktur 21 in Form eines Aussengewindes auf der Mantelfläche des Rotationskörpers 2 je 1,0 bis 2,0 mm über die Deck- und Grundfläche 11,12 aus dem Käfig 1 heraus.

In den Fig. 7 und 8 ist eine weitere Ausführungsform des erfindungsgemässen Implantats dargestellt, bei welcher der Käfig 1 zweiteilig und der Rotationskörper 2 als Kegelstumpf ausgebildet sind. Die Konstruktion des Implantats ist weitgehend identisch zu derjenigen der Ausführungsform nach den Fig. 1 - 6, mit Ausnahme des Käfigs 1, welcher aus zwei Einzelteilen, einem Oberteil la und einem Unterteil 1b besteht. Der Oberteil 1a ist mit vier Nocken 30 versehen, welche in die entsprechenden vier Ausnehmungen 31 im Unterteil 1b einrastbar sind, so dass der kegelstumpfförmige Rotationskörper 2 einfach und rasch zwischen den beiden Einzelteilen 1a,1b montiert und drehbar fixiert werden kann.

Der Käfig 1 und der Rotationskörper 2 sind vorzugsweise aus Titan, Titanlegierung, Keramik oder einem biokompatiblen Kunststoff gefertigt.

Nachstehend wird nun anhand des Implantats nach den Fig. 7 und 8 die klinische Anwendung im Detail beschrieben.
Der in Fig. 7 gezeigte, hohle Rotationskörper 2 wird mit Knochenspänen (bone graft oder Knochenersatzmaterial) - eventuell unter Komprimierung derselben - gefüllt und mit dem Verschlussdeckel 23 verschlossen. Danach wird der gefüllte Rotationskörper 2 zwischen die beiden Einzelteile 1a und 1b des Käfigs 1 gelegt, wobei der Zapfen 25 des Rotationskörpers 2 in die Ausnehmungen 26 der Einzelteile 1a,1b zu liegen kommt und durch leichte Druckausübung von oben und unten die beiden Einzelteile 1a und 1b, welche ein Einrasten der Nocken 30 in die Ausnehmungen 31 bewirken, zum Käfig 1 geschlossen werden. Das Implantat wird nun möglichst ohne Distraktionsinstrumente in den ausgeräumten Zwischenwirbelraum eingedreht. Ein Antriebswerkzeug - vorzugsweise ein Sechskantschraubenzieher - kann nun durch die Bohrung 17 in der Frontwand 13 des Käfigs 1 in die Sechskantöffnung 27 des Verschlussdeckels 23 eingeführt werden, so das sich der Rotationskörper 2 im Käfig 1 leicht drehen und dank seiner über dem Käfig 1 überstehenden spiralförmigen Struktur 21 in Form eines Gewindes in den Zwischenwirbelraum schrauben lässt.

## Patentansprüche

1. Zwischenwirbel-Implantat mit einem an seiner Deck- und Grundfläche (11,12) geöffneten rahmenförmigen Käfig (1) mit zwei Seitenflächen (13,14) einer Frontwand (15) und einer Hinterwand (16), dadurch gekennzeichnet, dass
A) ein Rotationskörper (2) drehbar im Käfig (1) gelagert ist, wobei die Mantelfläche des Rotationskörpers (2) mit einer spiralförmigen Struktur (21) versehen ist;
B) die spiralförmige Struktur (21) über die Deck- und Grundfläche (11,12) aus dem Käfig (1) herausragt; und
C) die Frontwand (15) eine Öffnung (17) zur Einführung eines Antriebswerkzeuges aufweist, mit welchem der Rotationskörper (2) im Käfig (1) rotiert werden kann.

2. Implantat nach Anspruch 1, dadurch gekennzeichnet, dass der Rotationskörper (2) hohl ausgebildet ist und an seiner Frontwand (22) vorzugsweise einen Verschlussdeckel (23) aufweist.

3. Implantat nach Anspruch 1 oder 2, dadurch gekennzeichnet, dass der Käfig (1) quaderförmig und der Rotationskörper (2) kreiszylinderförmig ausgebildet ist.

4. Implantat nach Anspruch 1 oder 2, dadurch gekennzeichnet, dass der Käfig (1) keilförmig und der Rotationskörper (2) konisch ausgebildet ist.

5. Implantat nach einem der Ansprüche 1 - 4, dadurch gekennzeichnet, dass der Rotationskörper (2) mit Perforationen (24) versehen ist, vorzugsweise in Form von Längsausnehmungen mit Schneidkanten.

6. Implantat nach einem der Ansprüche 1 - 5, dadurch gekennzeichnet, dass der Käfig (1) zweiteilig zusammensetzbar ausgebildet ist.

7. Implantat nach einem der Ansprüche 1 - 6, dadurch gekennzeichnet, dass Deck- und Grundfläche (11, 12) mit einer dreidimensionalen Strukturierung (18), vorzugsweise in Form von Längsrillen versehen ist.

8. Implantat nach einem der Ansprüche 1 - 7, dadurch gekennzeichnet, dass die Seitenflächen (13,14) mit Langlochausnehmungen (19) versehen sind.

9. Implantat nach einem der Ansprüche 1 - 8, dadurch gekennzeichnet, dass die spiralförmige Struktur (21) ein Aussengewinde, eine spiralförmige Rille oder Nut ist.

10. Implantat nach einem der Ansprüche 1 - 9, dadurch gekennzeichnet, dass die spiralförmige Struktur (21) 1,0 - 2,0 mm, vorzugsweise 1,35 - 1,70 mm über die Deck- und Grundfläche (11,12) aus dem Käfig (1) herausragt.

11. Implantat nach einem der Ansprüche 1 - 10, dadurch gekennzeichnet, dass die Steigung der spiralförmigen Struktur (21) mindestens 2 mm beträgt.

12. Implantat nach einem der Ansprüche 1 - 11, dadurch gekennzeichnet, dass die Tiefe der spiralförmigen Struktur (21) zwischen 0,8 - 2,2 mm, vorzugsweise zwischen 1,5 - 2,0 mm liegt.

## Claims

1. An intervertebral implant with a frame-like cage (1), which is open on its cover face and base face (11, 12), with two lateral faces (13, 14), with a front wall (15) and a rear wall (16), characterized in that
A) a rotation element (2) is rotatably mounted in the cage (1), the outer surface of the rotation element (2) here having a helical structure (21);
B) the helical structure (21) extends out of the cage (1) beyond the cover and base faces (11, 12); and
C) the front wall (15) has an aperture (17) so that a drive tool can be introduced, by means of which the rotation element (2) can be rotated in the cage (1).

2. The implant of Claim 1, wherein the rotation element (2) is designed hollow, and preferably has a sealing cover (23) on its front wall (22).

3. The implant of Claim 1 or 2, wherein the cage (1) has the shape of a rectangular parallelepiped, and the rotation element (2) has the shape of a circular cylinder.

4. The implant of Claim 1 or 2, wherein the cage (1) is designed wedge-shaped and the rotation element (2) is designed conical.

5. The implant of one of the Claims 1 - 4, wherein the rotation element (2) has perforations (24), preferably in the form of longitudinal recesses with cutting edges.

6. The implant of one of the Claims 1 - 5, wherein the cage (1) is designed so that it is composed of two parts.

7. The implant of one of the Claims 1 - 6, wherein the cover and base faces (11, 12) are equipped with a three-dimensional structure (18), preferably in the form of longitudinal grooves,

8. The implant of one of the Claims 1 - 7, wherein the lateral faces (13, 14) are equipped with longitudinal hole recesses (19).

9. The implant of one of the Claims 1 - 8, wherein the helical structure (21) is an outside thread, a helical groove or channel.

10. The implant of one of the Claims 1 - 9, wherein the helical structure (21) extends out of the cage (1), beyond the cover and base faces (11, 12), by 1.0 - 2.0 mm, preferably 1.35 -1.70 mm.

11. The implant of one of the Claims 1 - 10, wherein the pitch of the helical structure (21) is at least 2 mm.

12. The implant of one of the Claims 1 - 11, wherein the depth of the helical structure (21) lies between 0.8 and 2.2 mm, preferably between 1.5 and 2.0 mm.

## Revendications

1. Implant intervertébral présentant une cage (1) en forme de cadre, ouverte sur sa surface de couvercle et sa surface de base (11, 12), avec deux surfaces latérales (13, 14), une paroi frontale (15) et une paroi arrière (16), caractérisé en ce que
A) un corps rotatif (2) est monté à rotation dans la cage (1), la surface d'enveloppe du corps rotatif (2) étant dotée d'une structure (21) en spirale ;
B) la structure en spirale (21) déborde au-delà de la surface de couvercle et de la surface de base (11, 12) de la cage (1) ; et
C) la paroi frontale (15) présente une ouverture (17) pour l'insertion d'un outil d'entraînement avec lequel le corps rotatif (2) peut être tourné dans la cage (1).

2. Implant selon la revendication 1, caractérisé en ce que le corps rotatif (2) reçoit une configuration creuse et de préférence présente sur sa paroi frontale (22) un couvercle de fermeture (23).

3. Implant selon la revendication 1 ou 2, caractérisé en ce que la cage (1) est en forme de parallélépipède rectangle et en ce que le corps rotatif (2) est configuré en forme de cylindre circulaire.

4. Implant selon la revendication 1 ou 2, caractérisé en ce que la cage (1) est en forme de biseau et en ce que le corps rotatif (2) est configuré en forme de cone.

5. Implant selon l'une des revendications 1 à 4, caractérisé en ce que le corps rotatif (2) est doté de perforations (24) qui présentent de préférence la forme d'évidements allongés à bords tranchants.

6. Implant selon l'une des revendications 1 à 5, caractérisé en ce que la cage (1) est configurée en deux pièces aptes à être assemblées.

7. Implant selon l'une des revendications 1 à 6, caractérisé en ce que la surface de couvercle et la surface de base (11, 12) sont dotées d'une structuration tridimensionnelle (18) qui présente de préférence la forme de sillons longitudinaux.

8. Implant selon l'une des revendications 1 à 7, caractérisé en ce que les surfaces latérales (13, 14) sont dotées d'évidements (19) en trous oblongs.

9. Implant selon l'une des revendications 1 à 8, caractérisé en ce que la structure en spirale (21) est un filet extérieur, un sillon ou une rainure en spirale.

10. Implant selon l'une des revendications 1 à 9, caractérisé en ce que la structure en spirale (21) déborde de 1,0 à 2,0 mm, de préférence de 1,35 à 1,70 mm, de la surface de couvercle et de la surface de base (11, 12) de la cage (1).

11. Implant selon l'une des revendications 1 à 10, caractérisé en ce que le pas de la structure en spirale (21) est d'au moins 2 mm.

12. Implant selon l'une des revendications 1 à 11, caractérisé en ce que la profondeur de la structure en spirale (21) est comprise entre 0,8 et 2,2 mm, de préférence entre 1,5 et 2,0 mm.
